# EUROPEAN PATENT SPECIFICATION

(11) **EP 0 972 521 B1**
(45) Date of publication and mention of the grant of the patent: **29.01.2003**
(21) Application number: 99202497.6
(22) Date of filing: 09.08.1996
(51) Int. Cl.: A61K 38/21, C07K 14/57

(54) **Pharmaceutical composition containing stimulators of interferon-gamma**
PHARMAZEUTISCHE ZUSAMMENSETZUNG ENTHALTEND INTERFERON-GAMMA STIMULATOREN
Composition pharmaceutique contenant des stimulateurs de l'interferon gamma

(30) Priority: 18.08.1995 GB 9516967
(43) Date of publication of application: 19.01.2000
(62) Divisional of application: 96927141.0
(73) Proprietor: Renovo Limited, Manchester M13 9XX (GB)
(72) Inventor: Ferguson, Mark William James, High Peak, Derbyshire SK23 7PX (GB)
(74) Representative: Banford, Paul Clifford

(56) References cited:
- EP-A- 0 328 255
- WO-A-87/07842
- WO-A-92/14480
- WO-A-94/07497
- GRANSTEIN ET AL.: "The systemic administration of gamma interferon..." THE JOURNAL OF INVESTIGATIVE DERMATOLOGY, vol. 93, no. 1, July 1989 (1989-07), pages 18-27, XP000197306 New York, USA
- LORTAT-JACOB ET AL.: "Interferon-gamma, an antifibrogenic cytokine..." PATH. RES. PRACT., vol. 190, 1994, pages 920-922, XP000616388

## Description

The present invention concerns pharmaceutical preparations for promoting the healing of wounds or fibrotic disorders, in particular for promoting the healing of wounds or fibrotic disorders with reduced scarring, and for promoting the healing of chronic wounds.

By "wounds or fibrotic disorders" is meant any condition which may result in the formation of scar tissue. In particular, this includes the healing of skin wounds, the repair of tendon damage, the healing of crush injuries, the healing of central nervous system (CNS) injuries, conditions which result in the formation of scar tissue in the CNS, scar tissue formation resulting from strokes, and tissue adhesion, for example, as a result of injury or surgery (this may apply to e.g. tendon healing and abdominal strictures and adhesions). Examples of fibrotic disorders include pulmonary fibrosis, glomerulonephritis, cirrhosis of the liver, and proliferative vitreoretinopathy.

In particular, there is a lack of compositions for promoting the healing of wounds or fibrotic disorders with reduced scarring. Scar tissue formation, although providing mechanical strength to a healed wound, can be unsightly and may impair the function of the tissue.

This is particularly the case in wounds which result in scar tissue formation in the CNS, the scar tissue inhibiting the reconnection of severed or re-growing nerve ends, so significantly affecting their function.

There is also a lack of compositions for use in the treatment of chronic wounds, for example venous ulcers, diabetic ulcers and bed sores (decubitus ulcers), especially in the elderly and wheel chair bound patients. Such compositions may be extremely useful in patients where wound healing is either slow or in whom the wound healing process has not yet started. Such compositions may be used to "kick-start" wound healing and may then be used in combination with compositions (e.g. those of PCT/GB93/00586) which promote the healing of wounds or fibrotic disorders with reduced scarring. Hence not only may a chronic wound be healed, but it may be healed with reduced scarring.

According to the present invention there is provided an inhibitor of IFN-γ (Interferon-γ) for use in promoting the healing of wounds and fibrotic disorders with reduced scarring.

IFN-γ (Type II or immune interferon) is produced primarily by T lymphocytes upon mitogen or antigen stimulation (Trinchieri *et al*., 1985, Immunology Today, 6: 131). IFN-γ (both murine and human) exert their effects through specific, saturable, binding to a single class of high affinity receptors found on a variety of cells including fibroblasts, endothelial cells and monocytes/macrophages.

IFN-γ has been widely studied (see, for example, Kovacs, E.J., 1991, Immunology Today, 12(1): 17-23 - who states that IFN-γ decreases fibroblast proliferation and connective tissue production, i.e. inhibits scar tissue formation). Past studies of the effects of IFN-γ at wound sites have shown (Pittel, B. *et al*., 1994, Plastic and Reconstructive Surgery, 93: 1224-1235) that in studies on the effect of intralesional injection of IFN-γ to hypertrophic scars (an abnormal thickening of muscle), most (6/7) patients showed relief of symptoms, and all patients showed reduced lesion size during treatment, although there was no change in the total collagen content. Duncan *et al* (1985, J. Exp. Med., 162: 516-527) and Amento *et al* (1985, J. Clin. Invest., 76: 836-848) have shown that IFN-γ inhibits collagen types I and III and fibronectin synthesis by dermal and synovial fibroblasts and collagen type II by chondrocytes in a dose-dependent manner. Murray *et al* (1985, J. Immunol., 134: 1619-1622) have also shown that IFN-γ is involved in macrophage activation *in vivo*. Tamai *et al* (1995, J. Invest. Dermatol., 104:384-390) have shown that IFN-γ is involved in the regulation of metalloproteases (MMP) and tissue inhibitor of metalloproteases (TIMP) in *in vitro* cell culture. Various uses for IFN-γ and antagonists of same are proposed in EP 0304291, EP 0528469, WO 92/06115, WO 91/02005, WO 88/07869, EP 0328255, WO 92/14480, WO 87/07842, WO 94/07497, and Lorat-Jacobs, H. *et al*., 1994, Path. Res. Pract. 190:920-922.

It appears that IFN-γ is a multi-potent molecule with many actions depending on the conditions of the environment to which it is added. Several groups have reported decreased collagen synthesis *in vitro* on addition of IFN-γ to cultures, and Granstein *et al* (1989, J. Invest Dermatol., 93: 18-27) have shown inhibition of collagen deposition and hence healing with reduced scarring in wounds treated with IFN-γ. From these results, it appears that the treatment of sites (of wounds or fibrotic disorders) with IFN-γ would result in healing with reduced scarring.

Experiments undertaken (see 'Experimental' section below) have shown that, very surprisingly, the inhibition of IFN-γ actually promotes healing with reduced scarring, despite the teachings of the prior art.

The inhibitor may, for example, be a neutralising antibody. It may be a monoclonal antibody, a polyclonal antibody, a phage-derived antibody, a genetically engineered antibody (e.g. diabody), or antibody derived from a transgenic mouse.

Alternatively, the inhibitor may be anything which inhibits IFN-γ from interacting with its receptor (i.e. antagonises IFN-γ receptor activation) or which inhibits the receptor's activation. It may, for example, be a molecule which mimics the IFN-γ receptor binding sequence and which binds to the receptor but does not activate it, thereby competitively inhibiting the binding of IFN-γ to the receptor and inhibiting the activation of the receptor.

The inhibitor may be used in conjunction with a pharmaceutically acceptable carrier, diluent or excipient.

The inhibitor may be used in conjunction with a composition for promoting the healing of wounds or fibrotic disorders with reduced scarring.

The inhibitor may be used in conjunction with a composition for promoting the healing of chronic wounds.

Also provided according to the present invention is a method for promoting the healing of wounds or fibrotic disorders with reduced scarring comprising inhibiting IFN-γ.

The inhibition may be achieved by administering to a site an inhibitor of IFN-γ. By "site" is meant a site of wounding or fibrotic disorder. The inhibitor may be an inhibitor according to the present invention.

Between about 300 and about 30,000 IU IFN-γ may be inhibited.

The IFN-γ may be inhibited immediately prior to wounding/onset (by "onset" is meant the onset of a fibrotic disorder). It may be inhibited immediately after wounding/onset, although it may also be inhibited later, for example within approximately 3 or 7 days of wounding/onset.

The method may be used in conjunction with a method for promoting the healing of wounds or fibrotic disorders with reduced scarring.

The method may be used in conjunction with a method for promoting the healing of chronic wounds.

According to a further aspect of the present invention there is also provided a stimulator of IFN-γ for use in promoting the healing of chronic wounds..

The experiments (see 'Experimental' section below) have also shown that, very surprisingly, treatment of a site with IFN-γ actually promotes the deposition of collagen and healing with increased scarring and therefore may be used to promote the healing of chronic wounds.

By "stimulator" is meant anything which may stimulate (i.e. agonise) the quantity or efficacy of active IFN-γ at a site or the activation of the IFN-γ receptor. This may be IFN-γ itself or partially modified form of IFN-γ. A partially modified form of IFN-γ may, for example, have a longer half-life than IFN-γ. Alternatively, it may be an inhibitor of IFN-γ metabolism.

Partial modification may be by way of addition, deletion or substitution of amino acid residues. A substitution may for example be a conserved substitution. Hence a partially modified molecule be a homologue of the molecule from which it was derived. It may have at least 40%, for example 50, 60, 70, 80, 90 or 95%, homology with the molecule from which it is derived.

The stimulator may be used in conjunction with a pharmaceutically acceptable carrier, diluent or excipient.

The stimulator may be used in conjunction with a composition for promoting the healing of wounds or fibrotic disorders with reduced scarring.

The stimulator may be used in conjunction with a composition for promoting the healing of chronic wounds.

Also provided according to the present invention is a method for promoting the healing of chronic wounds comprising stimulating IFN-γ at a site. By "stimulating" is meant increasing the quantity or efficacy of active IFN-γ at a site or the activation of the IFN-γ receptor.

The stimulation may be achieved by administering to a site a stimulator of IFN-γ. The stimulator may be a stimulator according to the present invention.

Between about 7,500 and 15,000 IU IFN-γ may be administered to stimulate a site.

The IFN-γ may be stimulated immediately prior to wounding. It may be stimulated immediately after wounding, although it may also be stimulated later, for example within approximately 3 or 7 days or longer of wounding.

The method may be used in conjunction with a method for promoting the healing of wounds or fibrotic disorders with reduced scarring.

The method may be used in conjunction with a method for promoting the healing of chronic wounds.

The invention will be further apparent from the following example which shows, by way of example only, forms of inhibition of IFN-γ and promotion of healing with reduced scarring, and of promotion of healing of chronic wounds.

### EXPERIMENTAL

### Method

84 male CD1 mice, 12 to 15 weeks old (Charles River) were anaesthetised using equal parts halothane, oxygen and nitrous oxide. 2 x 1cm full-thickness incisions (through the panniculus carnosus) were made 3cm from the base of the skull and 1cm either side of the dorsal midline.

Test solutions used were anti-IFN-γ, IFN-γ and PBS. Anti-IFN-γ comprised monoclonal antibody against murine IFN-γ (MuIFN-γ; = rat IgG'2a). Antibodies were obtained as ascites fluid from thymusless nude-mice innoculated with the F3 hybridoma clone (J. Immunol., 1987, 138: 4178) and purified by affinity chromatography on an anti-rat kappa-chain mAb. The neutralisation potential of the antibody was 1/1,000,000 against 30U/ml of MuIFN-γ and contained 1.25 ng/ml endotoxin. IFN-γ was Chinese hamster ovary (CHO) cell-derived reconbinant MuIFN-γ purified by affinity chromatography on anti-IFN-γ mAb. The IFN-γ was at an initial concentration of 300,000 IU/ml (endotoxin: 73pg/ml).

Animals were split into several groups as follows:
- Group A:: Animals were treated with a single intraperitonal (IP) injection (100µl) of neat anti-IFN-γ antibodies prior to wounding.
- Group B:: Animals were treated with a single intradermal (ID) injection of 50µl or 25µl of anti-IFN-γ antibodies (diluted with PBS) prior to wounding.
- Group C:: Animals were treated with a single ID injection of IFN-γ (15,000 or 7,500 IU) prior to wounding.
- Group D:: Animals were treated with ID injections of IFN-γ (15,000 or 7,500 IU) on day 0 prior to wounding and days 3 and 7 post-wounding.
- Group E:: Animals were treated with a single control IP injection of PBS (phosphate buffered saline) on day 0 prior to wounding (control).
- Group F:: Animals were treated with a single control ID injection of PBS on day 0 prior to wounding.
- Group G:: Animals were treated with an ID injection of PBS on day 0 prior to wounding and days 3 and 7 post-wounding.

Animals were killed by chloroform overdose on days 7, 14, 70 & 120 post-wounding. Wounds were excised and bisected for routine histology and immunocytochemistry. 7µm wax sections were cut and stained for Haemotoxylin and Eosin to assess cell invasion and re-epithelialisation, and for Masson's Trichome to assess collagen deposition and orientation.

### Results

### Anti-IFN-γ antibodies:

No difference was observed between control wounds and treated wounds at any time point in the animals treated with a single IP injection.

With a single ID injection of anti-IFN-γ, there were no differences compared to controls at 7 and 14 days. However, by 70 and 120 days, marked differences in the orientation of the collagen fibres within the treated wound were observed.

Anti-IFN-γ treatment is anti-scarring, improving the quality of dermal architecture, despite the prior art observations. While the fibres were still relatively small and compacted immediately under the epidermis, they are randomly orientated, whereas in the mid and deep dermis the collagen fibres were less compacted and were orientated in a "basketweave" fashion. Control wounds (scarred) had compacted parallel collagen fibres throughout the wound area.

### IFN-γ

At the early time points (7 and 14 days), all the IFN-γ-treated wounds (in both injection regimes) showed increased inflammation and angiogenesis in a dose-dependent manner, i.e. lower doses, although worse than control wounds, were not as bad as wounds treated with higher doses of IFN-γ.

By 70 and 120 days, the wounds treated on days 0, 3 and 7 post-wounding with a high dose of IFN-γ showed marked fibrosis (i.e. scarring). Macroscopically, the wounds were raised and, microscopically, densely packed collagen in large swirling bundles within the wound margins was observed. These treated wounds also showed residual inflammation at the base of the wound, compared to control wounds. Again, this scarring was dose-dependent, i.e. the greater the dose of IFN-γ, the greater the scarring.

### Discussion

Previous work has shown that administration of IFN-γ to wounds inhibits collagen synthesis, suggesting that it may be useful as an anti-scarring agent. Other workers have shown that treatment of keloids or hypertrophic scars with IFN-γ decreases the size of the scar.

Contrary to these findings, these experiments have shown that, very surprisingly, the early treatment of wounds with IFN-γ causes fibrosis with raised scars that are packed full of collagen, whereas treatment of incisional wounds with antibodies to IFN-γ results in improved healing with collagen fibres orientated in a "basketweave" fashion resembling normal dermis (i.e. scarring is reduced).

## Claims

1. The use of a stimulator of IFN-γ in the manufacture of a medicament for promoting the healing of chronic wounds.

2. The use of a stimulator of IFN-γ according to claim 1 wherein it is selected from any one of the group of IFN-γ or a partially modified form thereof, and an inhibitor of IFN-γ metabolism.

3. The use of a stimulator of IFN-γ according to either one of claim 1 or 2 in conjunction with a pharmaceutically acceptable carrier, diluent or excipient.

4. The use of a stimulator of IFN-γ according to any one of claims 1-3 in conjunction with a composition for promoting the healing of wounds or fibrotic disorders with reduced scarring.

5. The use of a stimulator of IFN-γ according to any one of claims 1-4 in conjunction with a composition for promoting the healing of chronic wounds.

6. The use according to any one of claims 1 to 5, comprising administering to a site of wounding a stimulator of IFN-γ.

7. The use according to any one of claims 1-6 comprising the use of between 7,500 and 15,000 IU IFN-γ

8. The use according to any one of claims 1-7, comprising stimulating IFN-γ either immediately prior to wounding or immediately after wounding.

9. The use according to any one of claims 1-8 used in conjunction with a method for promoting the healing of wounds or fibrotic disorders with reduced scarring.

## Patentansprüche

1. Verwendung eines Stimulators für IFN-γ bei der Herstellung eines Medikaments zur Förderung der Heilung von chronischen Wunden.

2. Verwendung eines Stimulators für IFN-γ nach Anspruch 1, wobei es aus einem von der Gruppe von IFN-γ oder einer teilweise modifizierten Form davon und einem Inhibitor des IFN-γ-Metabolismus ausgewählt ist.

3. Verwendung eines Stimulators für IFN-γ nach einem der Ansprüche 1 oder 2 in Verbindung mit einem pharmazeutisch verträglichen Träger, Verdünnungsmittel oder Excipienten.

4. Verwendung eines Stimulators für IFN-γ nach einem der Ansprüche 1-3 in Verbindung mit einer Zusammensetzung zur Förderung der Heilung von Wunden oder Fibroseerkrankungen mit verminderter Narbenbildung.

5. Verwendung eines Stimulators für IFN-γ nach einem der Ansprüche 1-4 in Verbindung mit einer Zusammensetzung zur Förderung der Heilung von chronischen Wunden.

6. Verwendung nach einem der Ansprüche 1 bis 5, umfassend Verabreichung eines Stimulators für IFN-γ an eine Stelle der Verletzung.

7. Verwendung nach einem der Ansprüche 1-6, umfassend die Verwendung von zwischen 7500 und 15000 IU IFN-γ.

8. Verwendung nach einem der Ansprüche 1-7, umfassend die Stimulierung von IFN-γ entweder unmittelbar vor der Verletzung oder unmittelbar nach der Verletzung.

9. Verwendung nach einem der Ansprüche 1-8, verwendet in Verbindung mit einem Verfahren zur Förderung der Heilung von Wunden oder Fibroseerkrankungen mit verminderter Narbenbildung.

## Revendications

1. Utilisation d'un stimulateur de l'IFN-γ dans la fabrication d'un médicament pour activer la guérison de blessures chroniques.

2. Utilisation d'un stimulateur de l'IFN-γ selon la revendication 1, dans laquelle il est choisi parmi l'un quelconque du groupe constitué par l'IFN-γ ou une forme partiellement modifiée de celui-ci et un inhibiteur du métabolisme de l'IFN-γ.

3. Utilisation d'un stimulateur de l'IFN-γ selon l'une quelconque des revendications 1 et 2, conjointement avec un véhicule, diluant ou excipient pharmaceutiquement acceptable.

4. Utilisation d'un stimulateur de l'IFN-γ selon l'une quelconque des revendications 1 à 3, conjointement avec une composition pour activer la guérison de blessures ou de troubles fibreux avec une formation réduite de cicatrice.

5. Utilisation d'un stimulateur de l'IFN-γ selon l'une quelconque des revendications 1 à 4, conjointement avec une composition pour activer la guérison de blessures chroniques.

6. Utilisation selon l'une quelconque des revendications 1 à 5, comprenant l'administration d'un stimulateur de l'IFN-γ à un site de blessure.

7. Utilisation selon l'une quelconque des revendications 1 à 6, comprenant l'utilisation d'entre 7500 et 15000 UI d'IFN-γ.

8. Utilisation selon l'une quelconque des revendications 1 à 7, comprenant la stimulation de l'IFN-γ soit immédiatement avant une blessure soit immédiatement après une blessure.

9. Utilisation selon l'une quelconque des revendications 1 à 8, conjointement avec un procédé pour activer la guérison de blessures ou de troubles fibreux avec une formation réduite de cicatrice.
